# EUROPÄISCHE PATENTANMELDUNG

(11) **EP 1 988 394 A1**
(43) Veröffentlichungstag der Anmeldung: **05.11.2008**
(21) Anmeldenummer: 07107520.4
(22) Anmeldetag: 04.05.2007
(51) Int. Cl.: G01N 33/487

(54) **Messsystem mit verteilten Funktionen**

(71) Anmelder: F. Hoffmann-La Roche AG, 4070 Basel (CH); Roche Diagnostics GmbH, 68305 Mannheim (DE)
(72) Erfinder: Hoenes, Joachim, Zwingenberg 64673 (DE); Zimmer, Volker, Laumersheim 67229 (DE); Scherer, Joerg, Zuchwil 4528 (CH); Thoes, Bruno, 66287 Quierschied (DE); Werner, Karl, 69168 Wiesloch (DE)
(74) Vertreter: Hörschler, Wolfram Johannes

(57) **Zusammenfassung**

Analysesystem (300), ein Analysegerät und ein Teststreifenmagazin (134) umfassend, zur Bestimmung eines Analyten in einer Körperflüssigkeit, dadurch gekennzeichnet, dass das Analysesystem eine Gruppe (120) von wiederverwendbaren Komponenten enthält und eine weitere Gruppe (140) von Komponenten eines Wcgwcrfartikcls (Disposablc) enthält, von der an einer Trennstelle (302) elektronikfreie Komponenten (132, 134) abtrennbar sind. Kritische Schnittstellen (150, 160) werden bei der Herstellung des Gerätes zusammengefügt und geprüft.

## Beschreibung

### Stand der Technik

Seit geraumer Zeit sind auf dem Markt integrierte Systeme für die Blutzuckermessung erhältlich. Diese integrierten Systeme setzen sich zusammen aus einem Messgerät, einer in dieses einlegbaren Batterie sowie einem Teststreifenmagazin. Das Teststreifenmagazin kann scheibenförmig ausgebildet sein (Bayer Dex) beziehungsweise trommelförmig konfiguriert sein (AccuChek Compact). Die Teststreifenmagazine enthalten in der Regel Codiermittel, z. B. in Gestalt eines Barcodes, zur Übergabe von chargenspezifischen Informationen an das Messgerät, an dem das Teststreifenmagazin zum Einsatz kommt. Aus Kostengründen ist der in der Codierung enthaltene Informationsinhalt begrenzt. Elektronische Codiermittel, wie z. B. ROM-Keys, die einen hohen Informationsinhalt aufweisen und denen demzufolge eine entsprechend höhere Flexibilität innewohnt, werden nicht eingesetzt.

In neueren Entwicklungen sollen derartige Codiermittel, die einen hohen Informationsgehalt aufweisen, zum Einsatz kommen. Es ergibt sich daher das Problem, einerseits die Kosten zu reduzieren und andererseits das Problem des verbleibenden Elektronikschrottes in Zusammenhang mit der Hausmüllproblematik zu lösen.

Die sichere Ankopplung des Codiermittels an das Gerät stellt eine kritische Schnittstelle dar. Elektrische Kontakte zu einem ROM-Key oder einer Smart Card verlangen die Einhaltung von engen mechanischen Toleranzen und den Einsatz von vielfach wiederverwendbaren Kontakten. RFID-Chips lassen sich zwar kontaktlos lesen, führen aber im Endergebnis zu höheren Kosten und zu einem wesentlich höheren Energieverbrauch.

WO 2005/006985 A2 bezieht sich auf ein Analysegerät und ein Analyseverfahren für Körperflüssigkeiten. Das Analysegerät umfasst ein mit einer Aufnahme für den Eingriff einer Körperpartie versehenes Gehäuse sowie einen zwischen einer Freigabestelle und einer Wirkstellung bezüglich der Aufnahme bewegbares Widerlager für die Körperpartie. Ferner umfasst das Analysegerät ein in einem linearen Stechhub in die gegen das Widerlager anliegende Körperpartie einstechbares Stechorgan sowie ein Testband zur Applikation von aus der Körperpartie austretender Körperflüssigkeit. Das Testband ist in einer Testbandeinheit aufgenommen. Ferner umfasst das Analysegerät eine Detektionseinheit zur Untersuchung der auf einem Abschnitt des Testbandes applizierten Körperflüssigkeit.

WO 2004/047642 A1 bezieht sich auf ein Analysegerät für Körperflüssigkeiten, bei dem ein Teststreifenband vorgesehen ist, welches die Körperflüssigkeit aufnimmt. In einem Vorratsbereich wird ein unbenutzter Vorrat des Teststreifenbandes aufgenommen. In einem Speicherbereich wird der mit Körperflüssigkeit kontaminierte Teil des Teststreifenbandes gespeichert. In einem Expositionsbereich zwischen dem Vorratsbereich und dem Speicherbereich wird ein Bereich des Teststreifenbandes zur Aufnahme der Körperflüssigkeit angeboten, wobei der Expositionsbereich eine Spitze umfasst, an welcher das Testmedium der Körperflüssigkeit dargeboten wird.

WO 2004/056269 A1 bezieht sich ebenfalls auf ein Analysegerät für Körperflüssigkeiten. Das Analysegerät umfasst ein Teststreifenband, auf welches die Körperflüssigkeit aufgebracht wird. Das Teststreifenband umfasst ein Band und mit einem Testmedium versehene Abschnitte, wobei sich zwischen sukzessiven aufeinander folgenden Testmedienabschnitten ein von Testmedium freier Abschnitt erstreckt. Das Analysegerät umfasst ferner einen Bevorratungsbereich, mit einem Gehäuse, in welchem ein unkontaminierter, in Bandform vorliegender Teststreifenvorrat enthalten ist, wobei das Gehäuse eine Öffnung aufweist. Der Bevorratungsbereich umfasst darüber hinaus Dichteinrichtungen zum Verschließen der Öffnung gegen die Umgebung. Im Dichtzustand der Öffnung befindet sich ein vom Testmedium freier Abschnitt des Teststreifenbandes zwischen einer Oberfläche, bei der es sich typischerweise um eine Wand des Gehäuses handelt, und dem Dichtelement.

Bei den aus dem Stand der Technik bekannten Analysegeräten, insbesondere gemäß WO 2005/006985, baut das Analysegerät aufgrund der Integration des die Teststreifen aufnehmenden Magazins recht groß. Außerdem ist im Analysegerät eine Stechhilfe aufgenommen, was die Baugröße des Analysegerätes ebenfalls nachteilig beeinflusst.

Die Entwicklungstendenzen bei vom Anwender handhabbaren Analysegeräten verlaufen dahingehend, dass zukünftige Behälter für Testelemente als Bandkassetten realisiert werden. In den Kassetten sind die Testträger als Band aufgenommen, welches abschnittsweise mit einer ein- oder mehrlagigen Beschichtung für Glukosebestimmungen zum Beispiel versehen ist. Ferner sind in diesen Kassetten mechanische Elemente für den Transport der in Bandform vorliegenden Teststreifen, als auch Schlüssel insbesondere für die Austrittsöffnung des unverbrauchten Bandmaterials aus einer Vorratskammer innerhalb des Magazins vorgesehen. Bei der aus WO 2005/006985 A2 bekannten Lösung ist es erforderlich, das Magazin in das Analysegerät einzulegen, was nicht zuletzt zu der relativ groß bauenden Ausführung des Analysegerätes gemäß WO 2005/006985 A2 führt.

Bei nahezu allen Geräten, die aus dem Stand der Technik bekannt sind, wird die kritische Schnittstelle zwischen Disposable (darunter sind Teststreifen und Teststreifenmagazin zu verstehen) und Messgerät kundenseitig hergestellt. Der konstruktive Aufwand und die Kosten für diese Schnittstelle sind erheblich, ohne dass wirkliche Funktionssicherheit in allen Fällen gewährleistet werden könnte.

### Darstellung der Erfindung

Der Erfindung liegt die Aufgabe zugrunde, die Kopplung zwischen einem Analysegerät und einem Testelementemagazin zu vereinfachen, sicherer zu gestalten, Elektronikschrott zu vermeiden und durch eine Wiederverwertung Kosten zu sparen.

Erfindungsgemäß wird vorgeschlagen, eine Messeinheit zur Auswertung der mit einer menschlichen Körperflüssigkeit benetzten Testelemente in ein Magazin zu integrieren, in dem das Teststreifenmaterial bandförmig gespeichert ist. Die Messeinheit umfasst eine Optik, der eine Elektronik mit LEDs, Fotodioden und mindestens einem ASIC zugeordnet ist. Bevorzugt enthält der mindestens eine ASIC eine serielle Schnittstelle, die zum Beispiel an einer Rückseite des Gehäuses des Magazins angebracht sein kann, so dass das Analysegerät einfach auf die serielle Schnittstelle aufgesteckt werden kann, um an der seriellen Schnittstelle vorliegende aufbereitete Daten, zum Beispiel hinsichtlich einer Glukosemessung, abzunehmen. Durch das erfindungsgemäß vorgeschlagene, eine höhere Integrationsstufe aufweisende Magazin zur Aufnahme des in Bandform vorliegenden Teststreifenmaterials kann eine einfachere mechanische Ankopplung des Magazins an das Analysegerät erreicht werden. Über die serielle Schnittstelle, die entweder an der Rückseite des Gehäuses des Magazins oder an einer Seitenfläche von diesem ausgeführt sein kann, erfolgt eine robuste Signalübertragung durch ein serielles Protokoll zwischen dem eine höhere Integrationsstufe aufweisenden Magazin und dem mit diesen einfach verbindbaren Analysegerät. Damit ist die Grundlage für kleinere Bauformen sowohl für das Analysegerät als auch für das kassettenförmig ausgebildete Magazin gelegt. Aufgrund des Umstandes, dass das erfindungsgemäß vorgeschlagene, höher integrierte Magazin nicht mehr in das Analysegerät eingelegt oder eingeschoben werden muss, sondern einfach aufgesteckt werden kann, stehen für die Auslegung hinsichtlich der Baugröße größere gestalterische Freiheiten zur Verfügung.

Durch die erfindungsgemäß vorgeschlagene Lösung kann aufgrund der Integration der Elektronik in die Kassette auch mittels Feuchtesensoren die Feuchte innerhalb der Kammer des Magazins gemessen werden, in dem der unbenutzte Teststreifenvorrat auf einer Wickelspule gespeichert ist. Eindringende Feuchtigkeit beeinträchtigt die Langzeitverwendung des Teststreifenmaterials. Durch die erfindungemäß vorgeschlagene Lösung kann die Feuchte, die entweder herstellungsbedingt im Teststreifenmaterial enthalten ist, oder die über eine Öffnung während der Benutzung und des Ausschubs des Teststreifenmaterials aus der Vorratskammer in diese eindringt, ermittelt werden, was eine Vorhersage über die verbleibende Benutzungszeit des in der Vorratskammer bevorrateten Teststreifenmaterials gestattet. Damit ist das erfindungsgemäß vorgeschlagene, höher integrierte Magazin in der Lage, dem Anwender eine Information über die Qualität beziehungsweise die eventuell noch verbleibende Aufbrauchdauer für den unbenutzten Testmaterialvorrat zu übermitteln.

Das erfindungsgemäß vorgeschlagene, bandförmiges Testmaterial aufnehmende Magazin umfasst darüber hinaus eine lösbar mit dem das Teststreifenmaterial aufnehmenden Magazin verbindbare Platine, an der auch mindestens ein Energiespeicher aufgenommen ist. Wird die Platine am Gehäuse des Magazins, beispielsweise mittels Führungsschienen oder eines Bajonettverschlusses, aufgebracht, so zum Beispiel aufgeschoben, erfolgt die Herstellung einer elektrischen Verbindung zwischen dem an der Platine angeordneten Energiespeicher und der Elektronik, so zum Beispiel dem ASIC, innerhalb des Gehäuses des höher integrierten Magazins. An der Platine ist des Weiteren ein ROM-Key zur Kodierung angebracht. Der vorgeschlagenen Lösung folgend, kann die Platine samt ROM-Key und Energiespeicher vor der Entsorgung von der bandförmiges Teststreifenmaterial aufnehmenden Kassette getrennt werden. Während das kontaminierte Teststreifenmaterial in den Haushaltsmüll entsorgt werden kann, kann die Platine entweder im Rahmen einer Elektronikbauteilentsorgung entsorgt werden; bevorzugt ist die Möglichkeit, dass der Anwender die Platinen jeweils sammelt und einer Wiederverwertung zuführt. Damit wird das Teststreifenmaterial, das nicht mehr wiederbenutzbar ist, abgetrennt, und die wieder verwertbaren Komponenten, so zum Beispiel die Platine in den Materialkreislauf zurückgeführt und können wiederbenutzt werden.

### Zeichnung

Anhand der Zeichnung wir die Erfindung nachstehend eingehender beschrieben.

Es zeigt:
- Figur 1: ist ein Schema eines aus dem Stand der Technik bekannten Gerätes zu entnehmen (AccuChek Compact), bei dem die Elemente des Gerätes in einer Gruppe zusammengefasst sind und die Elemente eines Wegwerfartikels (Disposable) in einer anderen Gruppe zusammengefasst sind,
- Figur 2: ein Analysegerät, welches insgesamt als Wegwerfsystem ausgelegt ist, und
- Figur 3: eine schematische Darstellung des erfindungsgemäß vorgeschlagenen Messsystems mit verteilten Funktionen, wobei die Komponenten des Analysegerätes in einer Gruppe und die Komponenten eines erweiterten Wegwerfartikels (Disposable) in einer anderen gesonderten Gruppe zusammengefasst sind, wobei die Disposablegruppe aus zwei voneinander trennbaren Teilen aufgebaut ist.

### Ausführungsbeispiele

Unter Magazin wird nachfolgend ein Aufnahmeelement verstanden, in welchem eine Anzahl von Testelementen aufgenommen ist. Das Magazin kann kassettenförmig ausgestaltet sein, wobei die Bevorratung von Testelementen, zum Beispiel in Form von Wickelspulen mit einer Spule für unverbrauchte Testelemente und einer Spule für verbrauchte Testelemente dargestellt sein kann. Das Magazin kann des Weiteren auch stapelförmig ausgebildet sein, wobei einzelne Testelemente so zum Beispiel in Streifenform in vertikaler, horizontaler oder in relativ zueinander geneigter Anordnung aufgenommen sein können. Das Magazin kann des Weiteren auch als scheibenförmiger Körper beschaffen sein, an dessen Umfang einzelne Testelemente in Schlitzen aufgenommen sind, wobei ein Antrieb das scheibenförmig konfigurierte Magazin von Testelement zu Testelement weiter schaltet.

Die vorliegende Erfindung wird im Folgenden anhand eines Gerätes mit bandförmigem Testelementvorrat in Streifenform und optischer Auswertungsmöglichkeit beschrieben, ohne dass die Erfindung auf eine derartige Ausführungsform beschränkt werden soll. Die Erfindung lässt sich ebenso gut an einem elektrochemisch auswertbaren, streifenförmig ausgebildeten Testelement, welches in einem Stapelmagazin oder einer anderen Ausführungsform aufgenommen ist, implementieren.

Unter Testelementen wird nachfolgend ein medizinisches Verbrauchsmaterial verstanden, welches zum Beispiel in Streifenform im Magazin bevorratet ist und welches der Bestimmung eines Analyten in einer menschlichen Körperflüssigkeit, zum Beispiel Blutzucker, Laktat oder Cholesterin und dergleichen, dient. Unter Öffnung wird nachfolgend eine Austrittsöffnung des zum Beispiel in Teststreifenform vorliegenden medizinischen Verbrauchsmaterials aus dem Magazin verstanden, die zum Beispiel als eine mittels einer an das Gehäuse des Magazins angestellten Dichtung oder als Längsschlitz mit Dichtlippen ausgebildet sein kann, um einen unerwünschten Feuchtigkeitseintritt in das Innere des die Testelemente aufnehmenden Magazins zu verhindern.

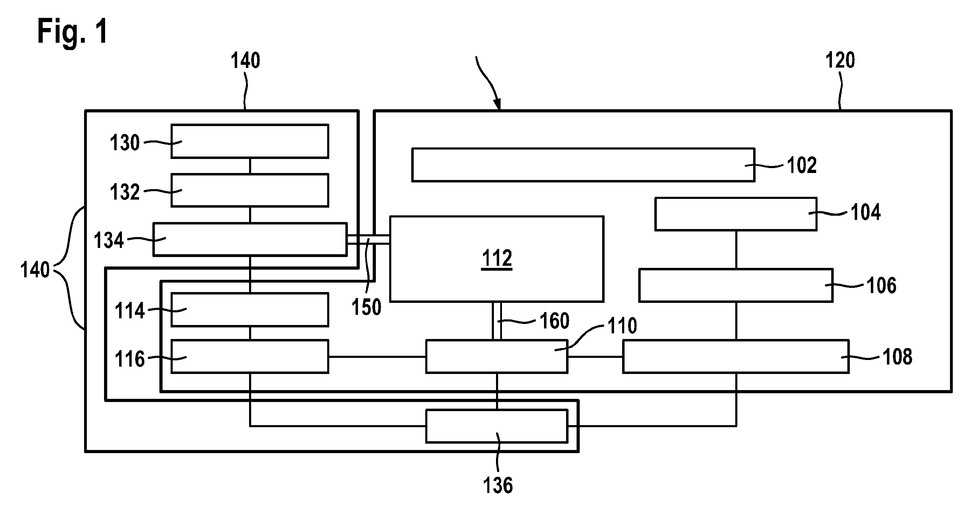

Der Darstellung gemäß Figur 1 ist ein aus dem Stand der Technik bekanntes Analysegerät zu entnehmen. Bei diesem Analysegerät handelt es sich z. B. um das System AccuChek Compact der Anmelderin. Das in Figur 1 in seinen wesentlichen Komponenten schematisch dargestellte Gerät 100 umfasst ein Gerätegehäuse 102, welches in der Regel als Kunststoffspritzgussteil hergestellt ist. Im Gerätegehäuse 102 befindet sich ein Display 104, auf welchem der Anwender erhaltene Ergebnisse ablesen kann, so z. B. Blutzuckergehalt, Cholesteringehalt und dergleichen mehr. Das in das Gerätegehäuse 102 integrierte Display 104 wird über eine Displaysteuerung 106 gesteuert. Die Displaysteuerung 106 ihrerseits steht mit einer mit Bezugszeichen 108 bezeichneten Berechnungselektronik in Verbindung, in der die auf dem Display 104 angezeigten Daten errechnet werden. Die Berechnungselektronik 108 des Gerätegehäuses 102 steht ihrerseits wiederum mit einer Messelektronik 110, die ebenfalls im Gerätegehäuse 102 aufgenommen ist, in Verbindung. Eine erste kritische Schnittstelle 150 verbindet Disposable (d. h. den Wegwerfteil) und das Messgerät miteinander. Die erste kritische Schnittstelle muss mit jedem neu eingesetzten unbenutzten Disposable in Kundenhand wieder hergestellt werden, was entsprechend enge Toleranzen und damit erhöhte Herstellkosten erfordert.

Die Messelektronik 110 ist über eine zweite kritische Schnittstelle 160 mit einer Messperipherie 112 verbunden. Innerhalb der Messperipherie des Gerätegehäuses 102 des Gerätes 100 gemäß der Darstellung in Figur 1 befinden sich zum Beispiel ein optisches Auswertesystem sowie andere elektrische Komponenten, wie z. B. ein Potentiometer sowie ein Amperemeter zur Ermittlung der Stromstärke. Die innerhalb der Messperipherie 112 im Gerätegehäuse 102 integrierten elektrischen Komponenten, von denen die obige Aufzählung nicht alle enthält, dienen der Auswertung eines in einem Teststreifenmagazin 134 bevorrateten Teststreifens, welcher mit einer zuvor erzeugten Öffnung in der menschlichen Haut z. B. mit einer Körperflüssigkeit wie Blut benetzt wird, welches danach im Wege eines chemischen oder optischen Verfahrens auf mindestens einen Analyten untersucht wird.

Darüber hinaus befindet sich innerhalb des Gerätegehäuses 102 ein Antrieb 114, der z. B. dem Vorschub von Teststreifen aus dem Teststreifenmagazin 134 dient. Dem Antrieb 114, bei dem es sich in der Regel um einen elektrischen Antrieb handelt, ist eine Antriebssteuerung 116 zugeordnet.

Aus der Darstellung gemäß Figur 1 geht hervor, dass die zuvor aufgezählten Komponenten, das Gerätegehäuse 102, das Display 104, dessen Displaysteuerung 106, die Berechnungselektronik 108, die Messelektronik 110, die Messperipherie 112 samt ihrer Komponenten Optik, Amperemeter, Potentiometer usw. sowie der Antrieb 114 sowie die Antriebssteuerung 116 sämtliche Elemente des Gerätes 100 bilden, die in der Darstellung gemäß Figur 1 in einer Gruppe 120 zusammengefasst sind.

Des Weiteren geht aus der Darstellung gemäß Figur 1 hervor, dass ein Codiermittel 130, ein Abfallbehälter 132, der der Aufnahme benutzter Teststreifen aus dem Teststreifenmagazin 134 dient, Komponenten eines Wegwerfartikels (Disposable) darstellen, der in der Darstellung gemäß Figur 1 durch Bezugszeichen 140 kenntlich gemacht ist. Der Energiespeicher 136 stellt einen weiteren separaten Energiespeicher dar. Hier stellen wie bei vielen anderen Geräten des täglichen Bedarfs die die Energiespeicher, bevorzugt als Batterien ausgebildet, kontaktierenden Batteriekontakte eine Quelle häufiger Störungen dar.

Bei der in Figur 1 dargestellten Lösung liegt die erste Schnittstelle 150 zwischen der Gruppe 140 der Komponenten des Wegwerfartikels (Disposable) und der Messperipherie 112 der Gruppe 120 der Komponenten des Gerätes, während die zweite kritische Schnittstelle 160 innerhalb der Gruppe 120, d. h. der Komponenten des Gerätes, liegt. Das Codiermittel 130, welches Bestandteil der Gruppe 140, d. h. Bestandteil des Wegwerfartikels ist, muss bei dieser Lösung sehr einfach sein und weist demzufolge einen nur beschränkten Informationsgehalt auf. Dies hat seine Ursache darin, dass das Codiermittel 130 ein Element der Gruppe 140, die den Wegwerfartikel (Disposable) charakterisiert, darstellt. Des Weiteren stellt der Energiespeicher 136, bei dem es sich in der Regel um eine Batterie handelt, einen weiteren Wegwerfartikel dar, der ein separates Verbrauchsteil darstellt und nicht kundenfreundlich ist. Zwar bietet die in Figur 1 skizzierte Lösung des Gerätes 100 den Vorteil, dass die meisten teuren, da höherwertigen Komponenten wiederverwendet werden, doch wiegt dieser Vorteil die aufgezählten Nachteile nicht auf.

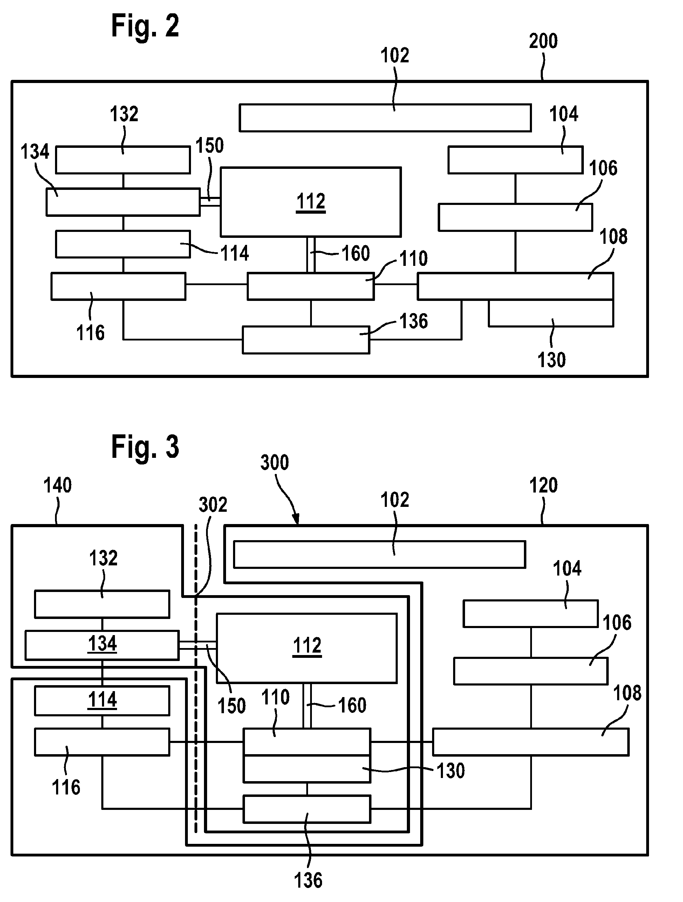

Der Darstellung gemäß Figur 2 ist eine weitere Ausführungsvariante eines Analysesystems zu entnehmen, das insgesamt als Wegwerfsystem konfiguriert ist.

Aus der Darstellung gemäß Figur 2 geht hervor, dass das Gerätegehäuse 102, das in diesem integrierte Display 104, die Displaysteuerung 106, die Berechnungselektronik 108 und das mit dieser zusammenwirkende Codiermittel 130, die Messelektronik 110, die Messperipherie 112, durch die zweite kritische Schnittstelle 160 miteinander verbunden, der Antrieb 114, die Antriebssteuerung 116, der Energiespeicher 136 sowie das Teststreifenmagazin 134, welches mit der Messperipherie 112 über die erste kritische Schnittstelle verbunden ist, samt Abfallbehälter 132 ein Wegwerfsystem 200 darstellen. Das in Figur 2 schematisch angedeutete Wegwerfsystem 200 hat den Nachteil, dass auch teure Komponenten, wie z. B. Elektronik und Display sowie Gerätegehäuse, nach einer Anzahl von Messungen weggeworfen werden, bietet jedoch den Vorteil, dass eine höhere Integration der Elektronikkomponenten möglich ist.

Der Darstellung gemäß Figur 3 ist das erfindungsgemäß vorgeschlagene Analysesystem mit einer neuverteilten Anordnung der Funktionen zu entnehmen, was nachfolgend den bekannten Ausführungsformen gemäß der Figuren 1 und 2 vergleichend gegenübergestellt wird.

Figur 3 zeigt, dass die Gruppe 120, welche die Elemente der Analysegerätes umfasst, das Gerätegehäuse 102, das Display 104, dessen Displaysteuerung 106, die Berechnungselektronik 108 und des Weiteren den Antrieb 114 und dessen Steuerung 116 umfasst. Dies bedeutet, dass teure Gerätekomponenten, wie z. B. der Antrieb 114, dessen Steuerung sowie das Display samt dessen Steuerung 106 sowie die Berechnungselektronik 108 weiterverwendet werden, wenn das in Figur 3 dargestellte Analysesystem mit verteilten Funktionen 300 mit einem neuen Teststreifenmagazin 134 bestückt wird.

Aus der Darstellung gemäß Figur 3 ist des Weiteren entnehmbar, dass die Gruppe 140, welche die Elemente des Wegwerfartikels (Disposable) enthält, den Abfallbehälter 132, das bereits erwähnte Teststreifenmagazin 134 sowie in diesem Falle die Messperipherie 112 und die Messelektronik 110, der ein Codiermittel 130 zugeordnet ist, samt Energiespeicher 136 umfasst. Diese Komponenten stellen die Elemente der Gruppe 140 dar, welche nach Verbrauch separat entsorgt werden können. Die in Figur 3 mit Bezugszeichen 140 bezeichnete Gruppe, welche die Elemente des Wegwerfartikels umfasst, schließt die erste kritische Schnittstelle 150 sowie die zweite kritische Schnittstelle 160 ein. Die erste kritische Schnittstelle 150 fällt im erfindungsgemäß vorgeschlagenen Analysesystem mit einer Trennstelle 302 zusammen, an welcher der Anwender das Teststreifenmagazin 134 samt diesem zugeordneten Abfallbehälter 132 mit der Messperipherie 112 verbindet. Die Trennstelle 302, die mit der ersten kritischen Schnittstelle 150 zusammenfällt, gestattet es in vorteilhafter Weise, die Gruppe 140, welche den Wegwerfartikel (Disposable) des erfindungsgemäß vorgeschlagenen Analysesystems darstellt, zu trennen. Die elektronikfreien Verbrauchsmaterialien, wie z. B. der Abfallbehälter 132 und das Teststreifenmagazin 134, können im Hausmüll entsorgt werden, während durch eine Auftrennung der Gruppe 140 an der Trennstelle 302 die Elektronikkomponenten, d. h. die Messperipherie 112, die mit dieser über die zweite kritische Schnittstelle 130 verbundene Messelektronik 110 samt dieser zugeordneter Codiermittel 130, wie insbesondere der Batteriespeicher 136, recycelt oder zumindest oder einer Elektronikschrottentsorgung zugeführt werden können, was bei dem in Figur 1 und 2 dargestellten Gerät 100 beziehungsweise Wegwerfsystem 200 gemäß der Darstellung in Figur 2 nicht möglich war.

Der erfindungsgemäß vorgeschlagenen Lösung gemäß der schematischen Darstellung in Figur 3 ist darüber hinaus entnehmbar, dass die erste und die zweite kritische Schnittstelle 150, 160 sich in einem bei dessen Herstellung verbundenen Bauteil, d. h. der Gruppe 140, befinden. Alle kritischen Schnittstellen werden herstellerseitig zusammengesetzt und kontrolliert. Die Funktionssicherheit erhöht sich bei geringeren Anforderungen an mechanische Toleranzen und damit bei fallenden, reduzierenden Herstellkosten.

Aufgrund der Ausgestaltung der Gruppe 140, welche die Elemente des Wegwerfartikels (Disposable) umfasst, mit der Trennstelle 302 ist es für den Anwender des in Figur 3 dargestellten Analysesystems problemlos möglich, die elektronischen Komponenten 112, 110, 136 der Gruppe 140 des Wegwerfartikels (Disposable) separat zu entsorgen oder diese einer Wiederverwertung zuzuführen. Die Trennung zwischen den in den Haushaltsmüll entsorgbaren Abfallbehälter 132 sowie das von Teststreifen entleerte Teststreifenmagazin 134 von den Elektronikkomponenten 110, 112, 130, 136 erfolgt an der Schnittstelle 302, was für den Anwender äußerst einfach und bequem ist.

Aus der schematischen Wiedergabe gemäß Figur 3 geht zudem hervor, dass das Codiermittel 130 der Messelektronik 110 direkt zugeordnet ist. Die direkte Zuordnung des Codiermittels 130 zur Messelektronik 110 bietet, um ein Beispiel zu nennen, als Vorteil, dass sich ein höherer Integrationsgrad erreichen lässt, d. h. eine geringere Baugröße des Gerätes bei Erhöhung der Funktionalitäten sowie eine Reduzierung der Kosten aufgrund der Vermeidung der Ausbildung anfälliger Steckverbindungen. Ferner besteht die Möglichkeit, Messelektronik und Messvorrichtung mit einem neuen Disposable an den neuesten Stand der Entwicklung anzupassen und dass ein Updaten von Messelektronik und Messvorrichtung über ein neues Disposable erfolgen kann, so dass das im Gebrauch befindliche Gerät 100 durch den Anwender weiterbenutzt werden kann. Hier stellt das Disposable dann das Medium dar, über das neue Informationen an das Gerät 100 gelangen.

Aus der Darstellung gemäß Figur 3 geht hervor, dass die Gruppe 140 der Komponenten des Wegwerfartikels das Codiermittel 130 umfasst. Im Vergleich zu den Lösungen gemäß des Standes der Technik, in der das Codiermittel 130 einen nur begrenzten Informationsgehalt hatte, kann das erfindungsgemäß in die Gruppe 140 des Wegwerfartikels (Disposable) integrierte Codiermittel 130 zum Beispiel als ROM-Key oder dergleichen ausgebildet werden, wodurch sich eine größere Flexibilität hinsichtlich des Informationsgehaltes ergibt. Bei elektronischen Speichermedien teilen sich die Kosten in der Regel in einem Verhältnis von 1/3 Speicherchip, 1/3 Gehäuse und 1/3 elektrische Kontakte auf. Durch die erfindungsgemäß vorgeschlagene Lösung kann man 2/3 dieser Kosten einsparen.

In Abwandlung der in Figur 3 dargestellten Gruppe 140, welche die Komponenten des Wegwerfartikels (Disposable) des erfindungsgemäß vorgeschlagenen Analysesystems mit verteilt angeordneten Funktionen darstellt, wäre auch denkbar, die Komponenten Abfallbehälter 132 beziehungsweise das entleerte und damit erschöpfte Teststreifenmagazin 134 von den Elektronikkomponenten 112, 110 und 136 entlang der Trennstelle 302 zu trennen und an die eventuell noch intakten Elektronikkomponenten 112, 110, 136 ein neues, mit einem frischen, unverbrauchten Vorrat bestückten Teststreifenmagazin 134 samt Abfallbehälter 132 aufzuwerten und eine derartige wiederverwendbare Gruppe 140 wieder innerhalb des Systems 300 mit verteilt angeordneten Funktionen einzusetzen.

Das in Figur 3 dargestellte System 300 mit verteilt angeordneten Funktionen erlaubt eine Wiederverwendung teurer Komponenten wie des Antriebes 114, der diesem zugeordneten Motorsteuerung 116, des Displays 104, der Displaysteuerung 106 sowie der Berechnungselektronik 108 samt Gerätegehäuse 102.

### Bezugszeichenliste

- 100: Gerät
- 102: Gerätegehäuse
- 104: Display
- 106: Displaysteuerung
- 108: Berechnungselektronik
- 110: Messelektronik
- 112: Messperipherie
- 114: Antrieb (Elektromotor)
- 116: Antriebssteuerung
- 120: Komponenten des Analysegerätes

- 130: Codiermittel
- 132: Abfallbehälter
- 134: Teststreifenmagazin
- 136: Energiespeicher (Disposable 2)

- 140: Komponenten eines Wegwerfartikels (Disposable 1)

- 150: erste kritische Schnittstelle
- 160: zweite kritische Schnittstelle

- 200: Wegwerfsystem

- 300: Analysesystem mit verteilt angeordneten Funktionen
- 302: Trennstelle (= erste kritische Schnittstelle)

## Patentansprüche

1. Analysesystem (300), ein Analysegerät und ein Teststreifenmagazin (134) umfassend, zur Bestimmung eines Analyten in einer Körperflüssigkeit, **dadurch gekennzeichnet, dass** das Analysesystem eine Gruppe (120) von wiederverwendbaren Komponenten enthält und eine weitere Gruppe (140) von Komponenten eines Wegwerfartikels (Disposable) enthält, von der an einer Trennstelle (302) elektronikfreie Komponenten (132, 134) abtrennbar sind.

2. Analysesystem (300) gemäß Anspruch 1, **dadurch gekennzeichnet, dass** die weitere Gruppe (140) von Komponenten eines Wegwerfartikels (Disposable) neben den elektronikfreien Komponenten (132, 134) Elektronikkomponenten (110, 112, 136) aufweist.

3. Analysesystem (300) gemäß Anspruch 1, **dadurch gekennzeichnet, dass** die weitere Gruppe (140) von Komponenten eines Wegwerfartikels (Disposable) eine Messelektronik (110) aufweist, in die ein Codiermittel (130) integriert ist.

4. Analysesystem (300) gemäß Anspruch 3, **dadurch gekennzeichnet, dass** das Codiermittel (130) als elektronischer Speicherchip ausgebildet ist.

5. Analysesystem (300) gemäß Anspruch 1, **dadurch gekennzeichnet, dass** eine erste kritische Schnittstelle (150) zwischen einer Messperipherie (112) und einem Teststreifenmagazin (134) innerhalb der weiteren Gruppe (140) von Komponenten eines Wegwerfartikels (Disposable) liegt.

6. Analysesystem (300) gemäß einem oder mehrerer der vorhergehenden Ansprüche, **dadurch gekennzeichnet, dass** eine zweite kritische Schnittstelle (160) zwischen der Messelektronik (110) und den Komponenten der Messperipherie (112) innerhalb der weiteren Gruppe (140) von Komponenten eines Wegwerfartikels (Disposable) liegt.

7. Analysesystem (300) gemäß einem der vorhergehenden Ansprüche, **dadurch gekennzeichnet, dass** mindestens eine kritische Schnittstelle (150, 160) zwischen
- dem Teststreifenmagazin (134) und der Messperipherie (112),
- der Messperipherie (112) und der Messelektronik (110) sowie der Messelektronik (110) und mindestens einem Energiespeicher
bei der Herstellung des Analysesystems (300) erzeugt und geprüft ist.

8. Analysesystem (300) gemäß Anspruch 1, **dadurch gekennzeichnet, dass** eine Messelektronik (110) und eine Messperipherie (112) bei Bestückung mit einer neuen, unverbrauchten, weiteren Gruppe (140) von Komponenten eines Wegwerfartikels aktualisierbar sind.

9. Analysesystem (300) gemäß Anspruch 1, **dadurch gekennzeichnet, dass** die Gruppe (120) von wiederverwendbaren Komponenten des Analysegerätes ein Gerätegehäuse (102) samt eines Displays (104), einer Displaysteuerung (106) sowie einer Berechnungselektronik (108) aufweist, die wiederverwendbar sind.

10. Analysesystem (300) gemäß einem oder mehrerer der vorhergehenden Ansprüche, **dadurch gekennzeichnet, dass** die Messperipherie (112) ein optisches Auswertesystem zur optischen Auswertung der im Teststreifenvorrat (134) bevorrateten, mit einer menschlichen Körperflüssigkeit benetzbaren Teststreifen enthält.

11. Analysesystem (300) gemäß einem oder mehrerer der vorhergehenden Ansprüche, **dadurch gekennzeichnet, dass** die Messperipherie (112) ein elektrochemisches Auswertesystem, insbesondere ein Amperemeter oder ein Potentiometer zur elektrochemischen Auswertung der im Teststreifenvorrat (134) bevorrateten, mit einer menschlichen Körperflüssigkeit benetzbaren Teststreifen, enthält.

12. Analysesystem (300) gemäß der Ansprüche 5 oder 6, **dadurch gekennzeichnet, dass** sich die erste kritische Schnittstelle (150) zwischen dem Teststreifenmagazin (134) und der Messperipherie (112) und die zweite kritische Schnittstelle (160) zwischen der Messperipherie (112) und der Messelektronik (110) sich auf einem bei der Herstellung verbundenen Bauteil befinden.

13. Analysesystem (300) gemäß einem oder mehrerer der vorhergehenden Ansprüche, **dadurch gekennzeichnet, dass** dieses mindestens einen Energiespeicher (136) aufweist, der Teil der weiteren Gruppe (140) von Komponenten eines Wegwerfartikels ist.

14. Analysesystem (300) gemäß Anspruch 13, **dadurch gekennzeichnet, dass** der mindestens eine Energiespeicher (136) einen separaten Wegwerfartikel außerhalb der weiteren Gruppe (140) von Komponenten eines Wegwerfartikels (Disposable) darstellt.
